# EUROPEAN PATENT APPLICATION

(11) **EP 4 349 975 A1**
(43) Date of publication of application: **10.04.2024**
(21) Application number: 21942233.4
(22) Date of filing: 25.05.2021
(51) Int. Cl.: C12N 7/01, C12N 15/24, C12N 15/23, C12N 15/19, C12N 15/34, A61K 35/761, A61K 38/20, A61K 38/21, A61K 38/19, A61K 48/00, A61P 35/00, A61P 35/04, C12R 1/93

(54) **CHIMERIC BROAD-SPECTRUM ONCOLYTIC ADENOVIRUS WITH MULTIPLE MECHANISMS SYNERGIZING WITH AND ENHANCING EFFICACY OF IMMUNOTHERAPY, AND APPLICATION THEREOF IN TUMOR TREATMENT**

(71) Applicant: Voncolytic Therapeutics Co., Ltd., Xuzhou, Jiangsu 221000 (CN)
(72) Inventor: LI, Jiang, Xuzhou, Jiangsu 221000 (CN); SU, Yinghan, Xuzhou, Jiangsu 221000 (CN)
(74) Representative: Eisenführ Speiser
(86) International application number: PCT/CN2021/095796
(87) International publication number: WO 2022/246646

(57) **Abstract**

Provided are a chimeric oncolytic adenovirus simultaneously expressing IL-12, IFN-γ, and CCL5, and an application thereof in tumor treatment. A capsid protein hexon of the oncolytic adenovirus is formed from chimerizing hexon sequences of the two serotype viruses Ad5 and Ad48, and a fiber protein is formed from chimerizing fiber sequences of the two serotype viruses Ad5 and Ad11. The chimeric oncolytic adenovirus can activate intrinsic anti-cancer activity of a variety of viral structural proteins, the ability to infect tumor cells is increased while also ensuring that the virus itself avoids interception from pre-existing neutralizing antibodies and adhesion and uptake of hepatocytes, and an effect of killing cancer cells is enhanced.

## Description

### FIELD

The present disclosure belongs to the fields of life sciences and biomedicine, and particularly relates to a chimeric broad-spectrum oncolytic adenovirus for synergistic and enhanced immunotherapy in tumor treatment via multiple mechanisms and use thereof in tumor treatment.

### BACKGROUND

Oncolytic virus-mediated gene therapy has high tumor cell killing efficiency, good specificity, high safety, few side effects, and low cost, making it an important emerging treatment option after the three conventional treatments (surgery, radiotherapy, and chemotherapy) and immunotherapy. The biggest challenge of oncolytic virus therapy is how to improve the safety and effectiveness of the product. Some safety regulatory mechanisms can ensure that the oncolytic virus replicates in tumor cells to a large extent but does not replicate in normal cells. Once the targeting specificity to tumors is improved, the safety is guaranteed. The mechanisms by which adenovirus targeting and killing tumor cells are diverse. For example, the early first-generation oncolytic adenovirus (OAV) achieved specific replication targeting P53 gene-deficient tumor cells by deleting the gene encoding E1b-55kD protein. The second-generation OAV uses various tumor-specific promoters to regulate the expression of the adenovirus proliferation gene E1a or/and E1b. The third-generation OAV uses a combination of regulatory elements. After several generations and diversified modifications, OAV's specificity and effectiveness against tumors have improved. Theoretically, OAV can serve as a vector for carrying anti-cancer genes, and can initiate viral replication as long as tumor cells are present. The anti-cancer genes will then be replicated in high copies and expressed with high efficiency, resulting in the dual tumor-killing effects of oncolysis by viral replication and expression of anti-cancer genes, without affecting normal cells. However, in practical applications, the tumor targeting specificity and tumor cell destruction effect of oncolytic viruses, including OAV, have not yet met people's expectations. The clinical effect of using oncolytic viruses alone to treat tumors is still not ideal. In addition, due to the limitations of differences in regulatory elements and selection of anti-cancer genes, OAV is highly individualized and difficult to produce good therapeutic effects in different tumors with large heterogeneity.

### SUMMARY

The purpose of the present disclosure is to provide a chimeric broad-spectrum oncolytic adenovirus for synergistic anti-cancer treatment via multiple mechanisms, wherein the oncolytic adenovirus can be used as an immune checkpoint inhibitor and a CAR-T/CAR-NK cell therapy synergist and use thereof in tumor treatment, in order to address the problem of poor effectiveness of existing tumor immunotherapy on solid tumors.

In a first aspect, the present disclosure provides a chimeric broad-spectrum oncolytic adenovirus for synergistic and enhanced immunotherapy via multiple mechanisms, wherein the oncolytic adenovirus simultaneously expresses three factors, IL-12, IFN-γ and CCL5; the capsid protein Hexon of the oncolytic adenovirus is a chimera of Hexon sequences of two serotype viruses, Ad5 and Ad48; the Fiber protein of the oncolytic adenovirus is a chimera of Fiber sequences of two serotype viruses, Ad5 and Ad11; the oncolytic adenovirus comprises a tumor-specific strong promoter and an oxygen-dependent element switch, and Elb-55kD gene is deleted, E1a-CR2, E1b-19kD and part of the protein coding sequence in E3 region are knocked out.

As a preferred embodiment of the present disclosure, in the genome of the oncolytic adenovirus, cDNA sequences of IFN-γ and CCL5 genes are in one expression cassette driven by human cytomegalovirus (hCMV) promoter, and cDNA sequence of IL-12 gene is in an expression cassette driven by the murine cytomegalovirus (mCMV) promoter, and the two expression cassettes are arranged "foot to foot".

More preferably, a sequence comprising the two expression cassettes simultaneously expressing three factors, IL-12, IFN-γ and CCL5 and the promoters is set forth in 29422-33108 bp of SEQ ID NO: 3.

As another preferred embodiment of the present disclosure, the capsid protein Hexon of the oncolytic adenovirus has a coding sequence set forth in 18327-21170 bp of SEQ ID NO: 3.

As another preferred embodiment of the present disclosure, the Fiber protein of the oncolytic adenovirus has a coding sequence set forth in 33373-34356 bp of SEQ ID NO: 3.

As another preferred embodiment of the present disclosure, the oncolytic adenovirus has an entire genome sequence set forth in SEQ ID NO: 3.

In a second aspect, the present disclosure provides an oncolytic adenovirus simultaneously expressing three factors, IL-12, IFN-γ and CCL5, wherein in the genome of the oncolytic adenovirus, cDNA sequences of IFN-γ and CCL5 genes are in one expression cassette driven by human cytomegalovirus (hCMV) promoter, and a cDNA sequence of IL-12 gene is in an expression cassette driven by the murine cytomegalovirus (mCMV) promoter, and the two expression cassettes are arranged "foot to foot".

As a preferred embodiment of the present disclosure, a sequence comprising the two expression cassettes simultaneously expressing three factors, IL-12, IFN-γ and CCL5 and the promoters is set forth in 29422-33108 bp of SEQ ID NO: 3.

In the third aspect, the present disclosure provides use of the above-mentioned chimeric broad-spectrum oncolytic adenovirus for synergistic and enhanced immunotherapy via multiple mechanisms. The present disclosure also provides use of the above-mentioned oncolytic adenovirus simultaneously expressing three factors, IL-12, IFN-γ and CCL5 in the manufacture of a medicament for the treatment and/or prevention and/or auxiliary treatment of a cancer or tumor.

As a preferred embodiment of the present disclosure, the cancer or tumor is selected from the group consisting of breast cancer, liver cancer, gallbladder cancer, gastric cancer, colon cancer, lung cancer, prostate cancer, lymphoma, colorectal cancer, ovarian cancer, cervical cancer, bile duct cancer, esophageal cancer, kidney cancer, glioma, melanoma, pancreatic cancer, bladder cancer and head and neck cancer.

The present disclosure provides the fourth generation OAV design solution based on 4 triple-construction techniques, and strives to improve the anti-cancer efficacy and biological safety of OAV to a new level as much as possible. The main features are as follows:
**1. Triple Transgene:** The chimeric broad-spectrum oncolytic adenovirus for synergistic and enhanced immunotherapy via multiple mechanisms, named VirRon, provided by the present disclosure, carries 3 anti-cancer genes in the E3 region of the adenovirus, including two immune-enhancing cytokine genes (IL-12 and IFN-γ) and one immune cell chemokine gene (CCL5), which establishes a direct and active molecular mechanism for the synergistic and enhanced OAV-combined immunotherapy. Using OAV as a vector to load anti-cancer genes can help specific high-copy replication and high-efficiency expression of anti-cancer genes in cancer cells. However, the selection of loaded anti-cancer genes is not just to meet the requirements of superimposed anti-cancer efficacy. A better solution is to establish a synergistic mechanism that can interact with the OAV itself, the tumor microenvironment, and the body's immune system. The suppressive state of the tumor microenvironment is the main obstacle to immunotherapy of solid tumors. Studies have confirmed that tumor cells infected with oncolytic viruses can release a large number of cytokines, and lysed tumor cells can also release a variety of tumor-related antigens. These cytokines and tumor-related antigens not only have immune activation effects, but can also change the tumor microenvironment, which enhances the effects of radiotherapy and chemotherapy, and also improves the effects of antibody therapy and immune cell therapy of immune checkpoint molecules (Oncoimmunology 2015, 4:e988098). However, the effect of existing oncolytic viruses on improving immunotherapy is limited and lacks a direct synergistic mechanism. The OAV provided by the present disclosure expresses three factors, IL-12, IFN-γ and CCL5, and has an active and direct synergistic mechanism to enhance immune checkpoint inhibitors and CAR-T/CAR-NK cell therapeutic efficacy. First, OAV is loaded with two immune-enhancing cytokines, IL-12 and IFN-γ. The treatment of PD-1 antibodies or PD-L1 antibodies requires activation of T cells to exert anti-cancer effects. This process is initiated by IFN-γ and IL-12, two cytokines synthesized and secreted by dendritic cells (DC), which communicate with T cells and then activate the killing activity of T cells (Immunity. 2018, 49:1148-1161.e7). However, DCs are often lacking in the tumor microenvironment, and the secretion of IFN-γ and IL-12 is insufficient. When OAV is loaded with immune-enhancing cytokines IL-12 and IFN-γ, DCs are not required to participate when OAV is combined with PD-1 antibodies. The IL-12 and IFN-γ expressed by the virus can directly activate killer T cells in the microenvironment and rapidly exert anti-cancer effects. When used in combination with CAR-T/CAR-NK, IL-12 expressed by OAV can prolong the survival time of CAR-T/CAR-NK cells, and IFN-γ can enhance the killing activity of CAR-T/CAR-NK. Indeed, IL-12 and IFN-γ themselves also have strong anti-cancer activity. IFN-γ is a well-established anti-cancer immune factor that can induce the expression of various cytokines such as interleukins and TNF. IL-12 is a new favorite in tumor immunotherapy, which can improve the body's protective immune response, promote the proliferation and activation of T cells and NK cells, and induce the secretion of IFN-γ and other cytokines. Secondly, OAV is loaded with the immune cell chemokine CCL5, which can participate in the chemotactic migration of NK, T cells (including CAR-T/CAR-NK), DC and other immune cells to accumulate in tumors (Cell 2018, 172:1022-1037). When OAV is combined with PD-1 antibodies, CCL5 can increase the number of NK, T cells, DC and other cells in the tumor microenvironment, and synergistically enhance the killing of cancer cells. When OAV is combined with CAR-T/CAR-NK, CCL5 can not only mediate the chemotaxis of more CAR-T/CAR-NK cells into the tumor area, but also promote chemotactic NK, T cells, DC and other cells to synergistically enhance CAR-T/CAR-NK function. Experiments have confirmed that the arrangement and connection order of the three factors in the oncolytic virus genome has a significant impact on its anti-tumor activity. By comparing the activity of oncolytic viruses with multiple combinations of the three factors that inhibit tumor cells, the order of the three factors in VirRon with the best anti-cancer activity was finally determined, that is: cDNA sequences of IFN-γ and CCL5 genes loaded by VirRon are in one expression cassette driven by human cytomegalovirus (hCMV) promoter, wherein the cDNA sequences of IFN-γ and CCL5 genes are connected by T2A; cDNA sequence of IL-12 gene loaded by VirRon is in an expression cassette driven by the murine cytomegalovirus (mCMV) promoter; and the two expression cassettes are arranged "foot to foot". The two expression cassettes respectively select two different human and mouse cytomegalovirus (CMV) promoters for driving, which can avoid unforeseen high-level structures caused by repetitions of the same sequence that affect the packaging success rate and anti-cancer activity of the virus.

The chimeric broad-spectrum oncolytic adenovirus for synergistic and enhanced immunotherapy via multiple mechanisms, named VirRon, provided by the present disclosure carries a reverse complementary sequence of the cDNA coding sequence of loaded IFN-γ gene (excluding the stop codon) set forth in 32263-32760 bp of SEQ ID NO: 3.

The chimeric broad-spectrum oncolytic adenovirus for synergistic and enhanced immunotherapy via multiple mechanisms, named VirRon, provided by the present disclosure carries a cDNA coding sequence of loaded IL-12 gene set forth in 29973-31583 bp of SEQ ID NO: 3.

The chimeric broad-spectrum oncolytic adenovirus for synergistic and enhanced immunotherapy via multiple mechanisms, named VirRon, provided by the present disclosure carries a reverse complementary sequence of the cDNA coding sequence of its loaded CCL5 gene set forth in 31924-32199 bp of SEQ ID NO: 3.

In the chimeric broad-spectrum oncolytic adenovirus for synergistic and enhanced immunotherapy via multiple mechanisms, named VirRon, provided by the present disclosure, the cDNA sequences of the loaded IFN-γ and CCL5 genes are in one expression cassette, and the cDNA sequences of IFN-γ and CCL5 genes are connected by T2A. The reverse complementary sequence of the T2A sequence is set forth in 32200-32262 bp of SEQ ID NO: 3.

In the chimeric broad-spectrum oncolytic adenovirus for synergistic and enhanced immunotherapy via multiple mechanisms, named VirRon, provided by the present disclosure, the cDNA sequences of the loaded IFN-γ and CCL5 genes are in one expression cassette driven by human cytomegalovirus (hCMV) promoter. The reverse complementary sequence of the coding sequence of the hCMV promoter is set forth in 32767-33108 bp of SEQ ID NO: 3.

In the chimeric broad-spectrum oncolytic adenovirus for synergistic and enhanced immunotherapy via multiple mechanisms, named VirRon, provided by the present disclosure, the cDNA sequence of IL-12 gene is in an expression cassette driven by the murine cytomegalovirus (mCMV) promoter. The sequence of the mCMV promoter is set forth in 29422-29944 bp of SEQ ID NO: 3.

For the chimeric broad-spectrum oncolytic adenovirus for synergistic and enhanced immunotherapy via multiple mechanisms, named VirRon, provided by the present disclosure, the anti-cancer genes are not limited to IL-12, IFN-γ and CCL5, but also include other cytokines, immune factors, tumor suppressor genes, suicide genes, antibody genes, etc. and their mutant sequences with anti-cancer activity. The connection of multiple genes is not limited to T2A sequence, but also includes F2A, P2A, IRES and other sequences and mutants thereof. The promoters used herein are not limited to mCMV and hCMV, but also include other promoters, enhancers and mutant sequences thereof with function of driving gene expression.

**2. Triple Chimerism:** The chimeric broad-spectrum oncolytic adenovirus for synergistic and enhanced immunotherapy via multiple mechanisms, named VirRon provided by the present disclosure, is a chimeric OAV of three serotypes, which is constructed by using the adenovirus Fiber coding sequence of subgenus B serotype Ad11, the adenovirus Hexon coding sequence of subgenus D serotype Ad48, and the adenovirus backbone sequence of subgenus C serotype Ad5. The oncolytic adenovirus achieves three advantages in one treatment system: improving the infection efficiency of the virus on tumor cells, preventing the virus from being intercepted by the body's pre-existing neutralizing antibodies, and reducing the adhesion and uptake of the virus by the body's liver cells. There are 52 serotypes in the human adenovirus family, divided into 6 subgenera (A to F). Except for group B, all groups of adenovirus use the coxsackievirus-adenovirus receptor (CAR) as their main recognition receptor, and their infection efficiency is very low for bone marrow hematopoietic cells, hematopoietic stem cells, dendritic cells, and some tumor cells, especially cancer stem cells, that lack CAR. Group B adenoviruses (Ad3, Ad7, Ad11, Ad14, Ad16, Ad21, Ad34-35, Ad50, Ad55, etc.) mainly recognize CD46, a widely expressed complement regulatory protein. Using the fiber knob of group B adenovirus to replace the fiber knob of Ad5 to construct a chimeric virus increases its ability to recognize the CD46 receptor, which is beneficial to improving the efficiency of the virus in infecting tumor cells, especially the ability to infect cancer stem cells, making it possible to more completely eradicate the root causes of tumor recurrence. Ad5 is widely distributed in nature, and most people have been infected and have produced neutralizing antibodies that can intercept viruses of the same serotype. Moreover, Ad5 is hepatotropic and can be adsorbed by liver cells. The hypervariable region (HVR) of the adenovirus capsid Hexon is exposed on the surface of the adenovirus, which is a key part that leads to differences in liver infection ability and immunogenicity between different serotypes of adenovirus. Selectively chimerizing the seven HVRs (L1: HVR1-6; L2: HVR7) in the Hexon molecule of Ad5 with the corresponding regions of the Hexon of rare serotypes such as subgroup D viruses (Ad8-10, Ad25-30, Ad36-39, Ad42-49, Ad51, etc.) is an effective method to help Ad5 evade pre-existing immunity and liver uptake. However, as Hexon is the main structural protein of adenovirus, its modification often results in an unstable structure of adenovirus vector, making it impossible to effectively package the virus. Therefore, this is a very challenging research. Through in-depth research in the early stage, we designed and combined multiple Hexon coding sequence fragments of Ad5 and Ad48 serotype viruses, and conducted virus recombinant packaging and amplification testing. We finally overcame this technical difficulty and successfully constructed multiple Hexon-replaced chimeric adenovirus vector skeletons, which can effectively package the target virus particles. The chimerism of Ad48 adenovirus Hexon can ensure that OAV evades the interception of the body's pre-existing neutralizing antibodies and the adhesion and uptake of adenovirus by liver cells.

In the chimeric broad-spectrum oncolytic adenovirus for synergistic and enhanced immunotherapy via multiple mechanisms, named VirRon, provided by the present disclosure, the sequence of the chimeric Fiber protein (Ad5F11b) in which the coding sequence of Fiber protein of subgenus B serotype Ad11 adenovirus is chimerized with the corresponding sequence of Fiber protein of subgenus C serotype Ad5 adenovirus is set forth in 33373-34356 bp of SEQ ID NO: 3.

In the chimeric broad-spectrum oncolytic adenovirus for synergistic and enhanced immunotherapy via multiple mechanisms, named VirRon, provided by the present disclosure, the sequence of the chimeric Hexon (Ad5H48) in which the coding sequence of Hexon of subgenus D serotype Ad48 adenovirus is chimerized with the corresponding sequence of Hexon of subgenus C serotype Ad5 adenovirus is set forth in 18327-21170 bp of SEQ ID NO: 3.

For the chimeric broad-spectrum oncolytic adenovirus for synergistic and enhanced immunotherapy via multiple mechanisms, named VirRon, provided by the present disclosure, its serotype chimerism is not limited to the chimerism of subgenus C Ad5 with subgenus B Ad11 and subgenus D Ad48, but also includes chimeric transformations of other serotypes of other subgenus.

**3. Triple Regulation:** The chimeric broad-spectrum oncolytic adenovirus for synergistic and enhanced immunotherapy via multiple mechanisms, named VirRon, provided by the present disclosure adopts a tumor-specific strong promoter and an oxygen-dependent element switch and deletes Elb-55kD gene to construct an OAV product with dual safety regulatory mechanisms at the transcription and translation levels. One of the mechanisms to achieve OAV's specific replication in tumor cells is to use tumor-specific promoters as cis-elements to control the expression of genes essential for adenovirus replication. By studying the molecular interaction mechanism between adenovirus E1a and E1b and cancer cells, we optimized the combination of regulatory modules in promoter and enhancer regulatory elements.

By studying the interaction mechanism between adenovirus E1a and E1b and cancer cell molecules, a preferable combination of regulatory modules is selected among promoter and enhancer regulatory elements. The tumor-specific broad-spectrum promoter is selected to control the expression of the adenovirus E1a gene only in tumor cells at the transcription level, and the E1a terminal is fused with an oxygen-dependent element switch. The E1a protein is protected in the hypoxic environment of tumors, but is degraded by the proteasome in normal tissue under normoxic conditions, effectively preventing the leakage of E1a protein expression in normal cells at the translation level. Elb transcription region encodes Elb-55kD and Elb-19kD proteins. Elb-55kD is a protein essential for adenovirus replication in normal cells but non-essential in tumor cells. The selective deletion of the gene encoding Elb-55kD can enable adenovirus to maintain its ability to replicate in tumor cells, but lose its ability to replicate in normal cells. Elb-55kD protein can inactivate and degrade P53 protein. The deletion of Elb-55kD helps cells maintain the anti-tumor activity of P53 while improving the targeting of viral vectors. Therefore, under the triple control of the above-mentioned regulatory mechanisms, the safety and effectiveness of VirRon are improved.

In the chimeric broad-spectrum oncolytic adenovirus for synergistic and enhanced immunotherapy via multiple mechanisms, named VirRon, provided by the present disclosure, the core sequence of the tumor-specific broad-spectrum promoter is derived from the "-857~+4 bp" fragment of the transcription start site of the 5'-UTR region of the BIRC5 gene. The full-length sequence is set forth in 467-1388 bp of SEQ ID NO: 3.

In the chimeric broad-spectrum oncolytic adenovirus for synergistic and enhanced immunotherapy via multiple mechanisms, named VirRon, provided by the present disclosure, the sequence of the oxygen-dependent element ON/OFF switch is set forth in 2376-2423 bp of SEQ ID NO: 3.

We have cloned, studied and preserved more than 20 tumor-specific gene promoters, enhancers and their mutant sequences in the early studies. For the chimeric broad-spectrum oncolytic adenovirus for synergistic and enhanced immunotherapy via multiple mechanisms provided by the present disclosure, the regulatory elements include the promoters, enhancers and mutant sequences derived from factors such as Ki67, hTERT, CEA, AFP, EGFR, DF3/MUC1, VEGFR, E2F, GFAP, Survivin, and also other regulatory sequences such as HIF-1 hypoxia response element (HRE), Egr-l radiation sensitive element (CArG), hTERT internal E-box, and oxygen-dependent switch (oxygen-dependent degradation domain, ODD), as well as combinations of the above sequences.

In the chimeric broad-spectrum oncolytic adenovirus for synergistic and enhanced immunotherapy via multiple mechanisms, named VirRon, provided by the present disclosure, the entire Elb-19kD coding sequence and the 1st to 437th codon of the Elb-55kD coding sequence in the Elb transcription unit were deleted, and the remaining Elb-55kD coding sequence is set forth in 2818-2997 bp of SEQ ID NO: 3.

**4. Triple Modification:** The chimeric broad-spectrum oncolytic adenovirus for synergistic and enhanced immunotherapy via multiple mechanisms, named VirRon, provided by the present disclosure simultaneously knocks out E1a-CR2, E1b-19kD and part of the protein coding sequence in E3 region. Based on serotype 5 (Ad5) of the human adenovirus C subgenus with the least pathogenicity, OAV is constructed by retaining the expression of E1a protein and making it regulated by a tumor-specific promoter to achieve tumor-specific replication. E1a has three functional regions, namely CR1, CR2 and CR3. The CR1 region can inhibit the expression of the Her-2/neu gene by binding to the transcription regulator P300/CBP. The CR2 region binds to the Rb protein family. The CR3 region is a transcriptional activation region. Therefore, the E1a protein itself has anti-tumor effects, not only by inhibiting the transcription of the Her-2/neu gene, blocking the activity of NF-κB, increasing the expression of p53, but also inhibiting the expression of protease genes such as type IV collagenase and plasminogen activator. E1a can also cause non-specific immune responses, increase the killing effect of CTL cells, NK cells and macrophages, induce tumor cell apoptosis, inhibit tumor invasion and metastasis, and improve the sensitivity of tumor cells to chemotherapy and radiotherapy. Introducing a 12-base deletion mutation into the CR2 region of E1a makes it unable to bind to the Rb protein and ensures that the dephosphorylated Rb protein forms a complex with the transcription factor E2F, which blocks the transcriptional activity of E2F, releases the intrinsic anti-cancer mechanism of E1a, and enhances the anti-cancer activity of the product. The Elb-19kDa gene is homologous to the apoptosis inhibitory gene Bcl-2, and the Elb-19kD protein can bind to Bax or/and Bak to initiate the downstream apoptosis inhibitory program and protect infected cells from TNF-α mediated killing. The deletion of Elb-19kD improves the specificity of the virus mutant's replication in tumor cells, while its replication activity in normal cells is weakened. The deletion of Elb-19kD can promote the restoration of the apoptosis pathway of cancer cells, and is conducive to the rapid clearance of the virus in normal cells and the rapid release and dissemination in tumor cells, making OAV better specific and more effective. The E3 transcription unit has 9 open reading frames and encodes proteins that protect infected cells from being killed by the host immune response. E3-gpl9k can weaken the CTL-mediated killing effect of infected cells. RID can block apoptosis mediated by "death" ligands including TNF, Fas ligand and TRAIL. RID can also inhibit the activation of NF-κB that is necessary for cell survival mediated by IL-1 and TNF. In addition to downregulating TRAIL receptors, E3-6.7k can also independently inhibit apoptosis induced by external and internal signaling pathways. E3-14.7k is a broad inhibitor of TNF-mediated apoptosis and can also bind to and inhibit Caspases-8 thereby preventing cell apoptosis initiated by the Fas signaling pathway. E3-14.7k interacts with FIP protein (for 14.7K-interacting protein, FIP-1, -2, -3) in infected cells, allowing E3-14.7k protein to play an important role in cell apoptosis and survival, inflammatory response, maintenance of membrane stability, nucleoplasmic transport and other signal transduction pathways. The molecular mechanism of the multifunctional E3 protein still needs to be further studied. Adenovirus death protein (ADP) can promote cell lysis and virus release, but the molecular mechanism remains unknown. It can be seen that deleting the E3 region while retaining the ADP gene during the OAV construction process can not only expand the vector capacity, but also promote the apoptosis of infected cancer cells, and is beneficial to promoting lysis of cancer cells by OAV and the release of progeny viruses.

In the chimeric broad-spectrum oncolytic adenovirus for synergistic and enhanced immunotherapy via multiple mechanisms, named VirRon, provided by the present disclosure, the 12 bases "cacgaggctggc" in E1a-CR2 are deleted. The oxygen-dependent element switch is inserted before the stop codon of the mutant E1a (mE1a) sequence, and its sequence is set forth in 1405-2426 bp of SEQ ID NO: 3.

For the chimeric broad-spectrum oncolytic adenovirus for synergistic and enhanced immunotherapy via multiple mechanisms provided by the present disclosure, the modification of viral structural proteins is not limited to the knockout of E1a-CR2, E1b-19kD and part of the protein coding sequence in E3 region, but also includes deletion, insertion, mutation and other modifications of E1a, E1b, E3 and other proteins.

To sum up, the chimeric broad-spectrum oncolytic adenovirus for synergistic and enhanced immunotherapy provided by the present disclosure is a new generation of OAV product that achieves the purpose of synergistic and enhanced effects via multiple mechanisms, with good specificity, strong anti-cancer activity and obvious therapeutic effect on a variety of solid tumors. It has overcome the previous problems of poor efficacy and narrow anti-tumor spectrum of oncolytic adenovirus used alone. It not only has good anti-cancer effects when used alone, but can also be used as an immune checkpoint inhibitor and a CAR-T/CAR-NK cell therapy synergist to greatly improve the efficacy on solid tumors in combination with immunotherapy. Its innovative significance is as follows:
**1. Multiple mechanisms precisely regulate OAV replication to improve its safety and effectiveness.** The tumor-specific broad-spectrum promoter is used to control the expression of adenovirus replication gene E1a, which enhances the specific replication activity of OAV against tumor cells at the transcriptional level. An oxygen-dependent element switch is fused inside the E1a protein to ensure that E1a is degraded under normoxic conditions in normal tissues and restricts virus replication in normal cells at the translation level. The selective deletion of Elb-55kD can enable adenovirus to maintain its ability to proliferate and replicate in tumor cells, but lose its ability to replicate in normal cells. The deletion of Elb-19kD can promote the restoration of the apoptosis pathway of cancer cells, and is conducive to the rapid clearance of the virus in normal cells and the rapid release in tumor cells. Knocking out part of the encoded protein in the E3 region and retaining the ADP gene achieve the purpose of protecting the long-term survival of adenovirus and mediating the high expression of anti-cancer genes. Multiple mechanisms work together to improve the safety and effectiveness of OAV
**2. The activation of the intrinsic anti-tumor mechanism of the virus synergistically improves the oncolytic effect and anti-cancer efficacy of OAV.** The adenovirus E1a protein itself has anti-tumor effects, not only by inhibiting the transcription of the Her-2/neu gene, blocking the activity of NF-κB, increasing the expression of p53, but also inhibiting the expression of protease genes such as type IV collagenase and plasminogen activator. E1a can also cause non-specific immune responses, increase the killing effect of CTL cells, NK cells and macrophages, induce tumor cell apoptosis, inhibit tumor invasion and metastasis, and improve the sensitivity of tumor cells to chemotherapy and radiotherapy. On this basis, introducing deletion mutation into the CR2 region of E1a makes it unable to bind to the Rb protein and ensures that the dephosphorylated Rb protein forms a complex with the transcription factor E2F, which blocks the transcriptional activity of E2F and can enhance the anti-cancer activity. In particular, OAV infects cancer cells to release a large amount of cytokines, and lyse cancer cells to release a large amount of tumor-related antigens, which exert further immune activation effect. Viral proteins expressed in cancer cells, similar to tumor-specific antigens, can also function as anti-cancer vaccines.
**3. OAV formed by chimerization of three serotypes adenoviruses increases its infectivity and can effectively evade immune interception and liver uptake.** The chimera of the fiber knob of group B adenovirus with the fiber knob of Ad5 is beneficial to improving the virus's infection efficiency on cancer cells, especially cancer stem cells. The selective chimera of the HVR region of the Hexon molecule of Ad5 with the corresponding region of the Hexon of subgroup D virus helps the virus evade interception of human neutralizing antibodies and uptake by liver cells.
**4. A treatment strategy for immune regulation of the tumor microenvironment for synergistic and enhanced effects via multiple mechanisms is established and obtains clear efficacy in research.** Using OAV as a vector to load anti-cancer genes helps specific high-copy replication and high-efficiency expression of anti-cancer genes in cancer cells as the virus replicates. By expressing anti-cancer genes or immunomodulatory genes that can activate immunity in the tumor microenvironment, efficient new OAV products with multiple mechanisms for synergistic anti-cancer effects can be developed, which can have synergistic effects with multiple treatments such as immune checkpoint inhibitor therapy and immune cell therapy, achieve multiple major breakthroughs in improving the safety and effectiveness of the virus, and achieve broad-spectrum, specific, safe and efficient anti-cancer effects.

### BRIEF DESCRIPTION OF DRAWINGS

FIG. 1 shows the structure of adenovirus right arm backbone plasmid pPE3F11bH48-RC(+).
FIG. 2 shows the structure of adenovirus left arm shuttle plasmid pAdSVP-mE1aODD.
FIG. 3 shows the structure of exogenous gene shuttle plasmid pENTR12-C3.
FIG. 4 shows the structure of the genome of oncolytic adenovirus VirRon.
FIG. 5 shows that oncolytic virus VirRon has high replication activity in cell lines of liver cancer, breast cancer, gallbladder cancer, and lung cancer with a replication multiple between 10⁴ and 10⁶ at 96 h after infection, and weak replication activity in normal cell lines.
FIG. 6 shows the curve of the cell killing effect of oncolytic virus VirRon, showing that VirRon has a significant killing and inhibiting effects on a variety of cell lines derived from solid tumors at a low MOI value, but has no killing activity on BJ cells (normal fibroblast cells).
FIG. 7 shows the comparison of the IC50 values of oncolytic virus VirRon on cell killing, showing that the IC50 value of killing and inhibiting effects on various solid tumor cells is low, while the IC50 value of the killing effect on BJ cells (normal fibroblast cells) is up to 1091 pfu/cell.
FIG. 8 shows that oncolytic virus VirRon expresses three anti-cancer genes "IL-12+IFN-y+CCL5", and the expression level gradually increases over time.
FIG. 9 shows that the arrangement and connection order of the three anti-cancer genes in oncolytic adenovirus VirRon affect its packaging efficiency and anti-cancer activity.
FIG. 10 shows that the combinations of chimeric Hexon (Ad5H48) fragments in oncolytic adenovirus VirRon affect its packaging and amplification efficiency.
Figure 11 shows the experiment results of oncolytic virus VirRon against liver cancer in nude mouse HCCLM3 transplanted tumors model.
Figure 12 shows the experiment results of oncolytic virus VirRon against breast cancer in nude mouse MDA-MB-231 transplanted tumors model.
Figure 13 shows the experiment of oncolytic virus VirRon against gallbladder cancer in nude mouse SGC996 transplanted tumors model.
Figure 14 shows the experiment results of VirRon combined with PD-1 antibody in treating humanized mouse model with colon cancer transplanted tumors.
Figure 15 shows the experiment results of VirRon combined with CAR-T cells in treating NCG mouse model with prostate cancer transplanted tumors.

### DETAILED DESCRIPTION

The specific embodiments provided by the present disclosure will be described in detail below in conjunction with the accompanying drawings.

### Example 1 Development of VirRon, the chimeric broad-spectrum oncolytic adenovirus for synergistic and enhanced immunotherapy via multiple mechanisms of the present disclosure

VirRon, the chimeric broad-spectrum oncolytic adenovirus for synergistic and enhanced immunotherapy via multiple mechanisms of the present disclosure, was constructed from an adenovirus right arm plasmid, an adenovirus left arm plasmid and an exogenous gene shuttle plasmid. The three plasmids and the recombination process are illustrated as follows:

### (1) Adenovirus right arm backbone plasmid

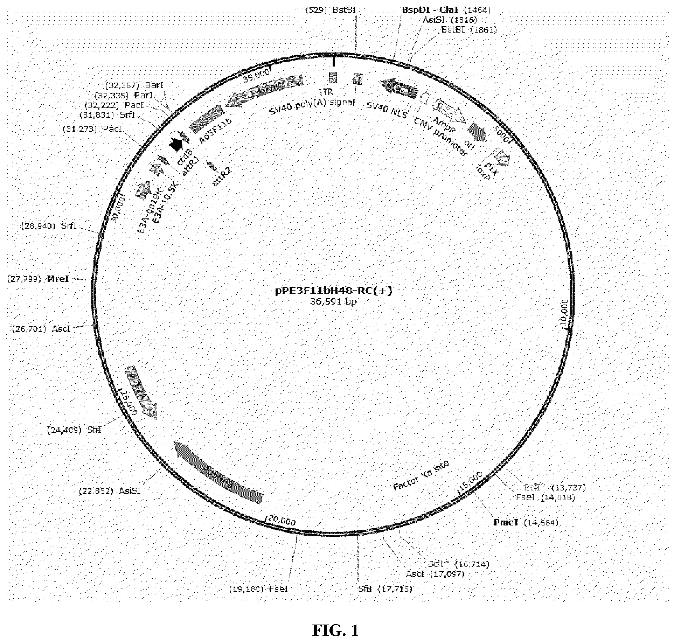

The adenovirus right arm backbone plasmid pPE3F11bH48-RC(+) was modified from the pBHGlox (delta) E13Cre plasmid (Catalog no. PD-01-40) and pBHGE3 plasmid (Catalog no. PD-01-12) provided by Microbix Biosystems of Canada. The fragment of pBHGE3 containing part of the E3 region sequence digested by SpeI + NotI and the synthetic ADP gene sequence (as shown in 30901-31182 bp of SEQ ID NO: 3) were inserted into the SpeI + PacI site of pBHGlox (delta) E13Cre to construct pPE3 plasmid. The synthetic attR1+ccdB+attR2 sequence (949 bp, SEQ ID NO: 1) was inserted into the PacI site of pPE3. The Ad5-Fiber sequence was replaced with the synthetic Ad5F11b fragment, and the Ad5-Hexon sequence was replaced with the synthetic Ad5H48 fragment. The pPE3F11bH48-RC(+) plasmid was finally constructed (FIG. 1). The full length of pPE3F11bH48-RC (+) plasmid is 36591 bp. The fiber protein (Ad5F11b) is a chimera of the corresponding sequences of Ad5 and Ad11 serotype adenoviruses (as shown in 33373-34356 bp of SEQ ID NO: 3), and the hexon protein (Ad5H48) is a chimera of the corresponding sequences of Ad5 and Ad48 serotype adenoviruses (as shown in 18327-21170 bp of SEQ ID NO: 3).

### (2) Adenovirus left arm shuttle plasmid

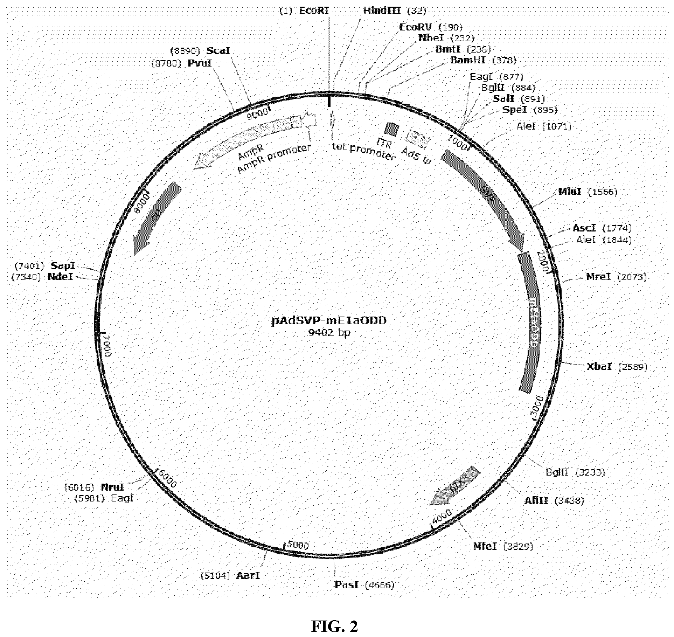

The adenovirus left arm shuttle plasmid pAdSVP-mE1aODD was modified from the p XC1 plasmid (Catalog no. PD-01-03) provided by Microbix Biosystems of Canada. A BglII restriction site was introduced 12 bp upstream of the E1a start codon of pXC1 to construct pXC2. The "-857~+4 bp" fragment of the transcription start site in the 5'-UTR region of the BIRC5 gene and the mutant E1a and the oxygen-dependent element switch sequence (mE1a-ODD), a total of 2355 bp (as shown in 467-2821 bp of SEQ ID NO: 3), were synthesized and inserted between the two BglII sites of pXC2 to replace the E1a and E1b sequences in pXC2. The pAdSVP-mE1aODD plasmid was then constructed, with a full length of 9402 bp (FIG. 2).

### (3) Exogenous gene shuttle plasmid

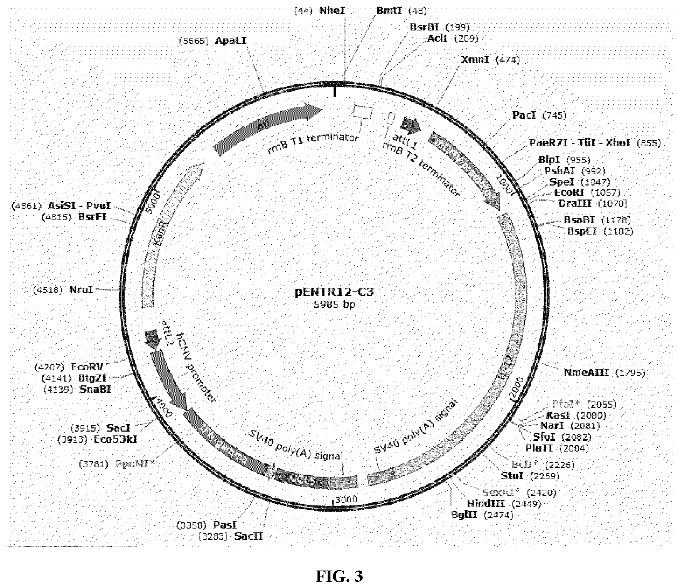

The shuttle plasmid pENTR12 was modified from the pENTR11 plasmid (Catalog no. 11819-018) provided by Invitrogen Company of the United States. The sequence of "mCMV promoter + SpeI/ EcoRI/ HindIII/ BglII/ NheI/ BamHI/ SalI+ Poly(A)" (SEQ ID NO: 2) was introduced between the NcoI and EcoRV restriction sites of pENTR11 to replace the multiple cloning site and ccdB gene sequence in the original pENTR11 plasmid to construct a new plasmid pENTR12. The IL-12 expression cassette and IFN-y+CCL5 expression cassette were inserted between the EcoRI and EcoRV multiple cloning sites of pENTR12, and the two expression cassettes were arranged "foot to foot" to construct the exogenous gene shuttle plasmid pENTR12-C3, a total of 5985 bp (FIG. 3). The complete sequence (including the site sequence) containing the IL-12 expression cassette and the IFN-y+CCL5 expression cassette between the NcoI and EcoRV sites that was finally transferred from pENTR12-C3 to the VirRon genome is as shown in 29401-33114 bp of SEQ ID NO: 3.

### (4) Recombinant packaging of VirRon virus

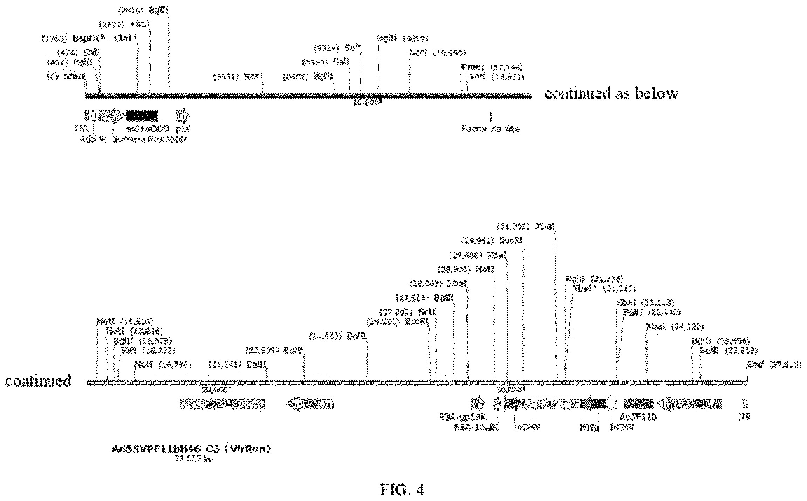

VirRon virus was packaged through two rounds of specific recombination with the adenovirus right arm plasmid pPE3F11bH48-RC(+) as the backbone. First, the exogenous gene shuttle plasmid pENTR12-C3 and the adenovirus right arm backbone plasmid pPE3F11bH48-RC(+) were subj ected to the first round of attL/attR site-specific recombination in E. coli competent cells DB3.1. The exogenous gene expression cassette in pENTR12-C3 was transferred into the E3 region of pPE3F11bH48-RC(+). Then the adenovirus left arm shuttle plasmid pAdSVP-mE1aODD and the product of the first round of recombination were subj ected to the second round of sequence-specific recombination in 293T cells, to transfer the mElaODD expression cassette controlled by the tumor-specific promoter in pAdSVP-mE1aODD into the E1 region of the adenovirus right arm backbone plasmid. After two rounds of specific recombination, the ideal oncolytic adenovirus VirRon was accurately and quickly packaged. The structure of oncolytic adenovirus VirRon is shown in FIG. 4. The sequence of VirRon, the chimeric broad-spectrum oncolytic adenovirus for synergistic and enhanced immunotherapy via multiple mechanisms of the present disclosure, is shown in SEQ ID NO: 3.

### Example 2 Cytological experiments using VirRon, the chimeric broad-spectrum oncolytic adenovirus for synergistic and enhanced immunotherapy via multiple mechanisms of the present disclosure

VirRon, the chimeric broad-spectrum oncolytic adenovirus for synergistic and enhanced immunotherapy via multiple mechanisms of the present disclosure, can specifically replicate in tumor cells, mediate the high-efficiency expression of anti-cancer genes, and finally destroy or inhibit tumor cells.

### (1) Specific replication of oncolytic adenovirus VirRon

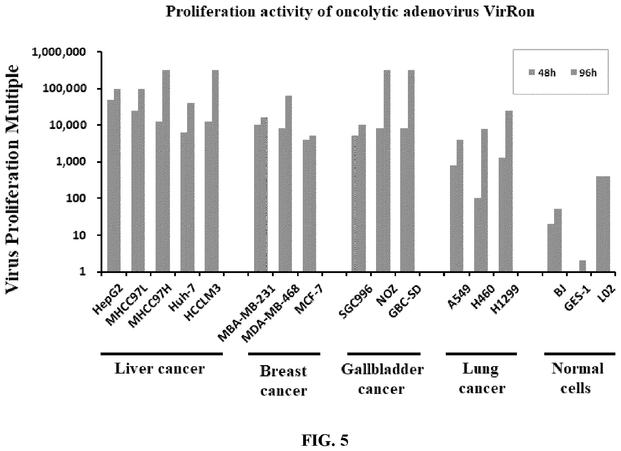

The solid tumor cell lines and normal cell lines in the logarithmic growth phase were collected and plated on a 96-well plate at a density of 1×10⁴ cells/well. After the cells adhered to the wall, serum-free culture medium was used instead. VirRon was added at MOI = 5 pfu/cell for cell infection. After 2 h of virus infection, culture medium containing 5% serum was used instead. After culturing for 0 h, 48 h, and 96 h, cells and supernatants at time points of 0 h, 48 h, and 96 h were collected. The virus titer was detected by the TCID50 method. Based on 0 h, the virus replication multiple was calculated. The results showed that VirRon exhibited high copy proliferation in most solid tumor cell lines including the cell lines derived from liver cancer, breast cancer, gallbladder cancer and lung cancer, with the replication multiple up to 316978.64 times. In contrast, the replication activity in normal cell lines was low, and there was no replication at all in GES-1 cells, and weak replication activity in hepatocyte L02 with a replication multiple of only 399.05 times (FIG. 5).

### (2) Specific killing and inhibition on tumor cells by oncolytic adenovirus VirRon

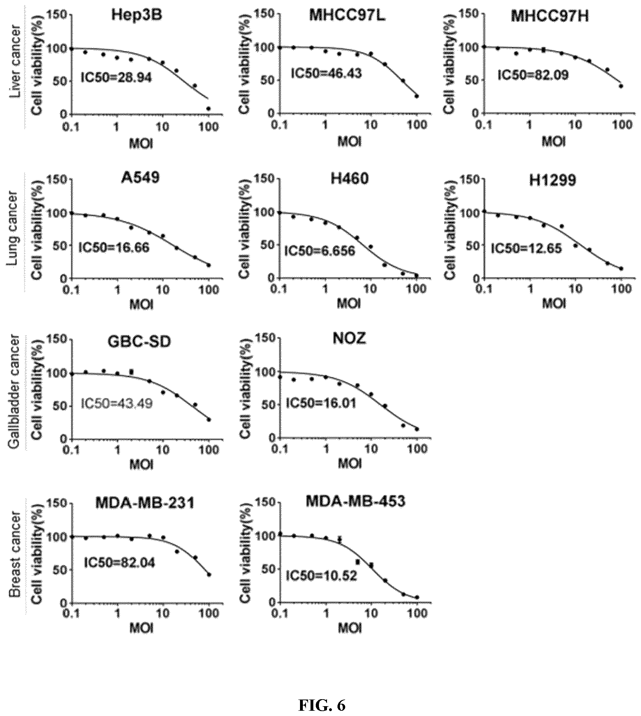

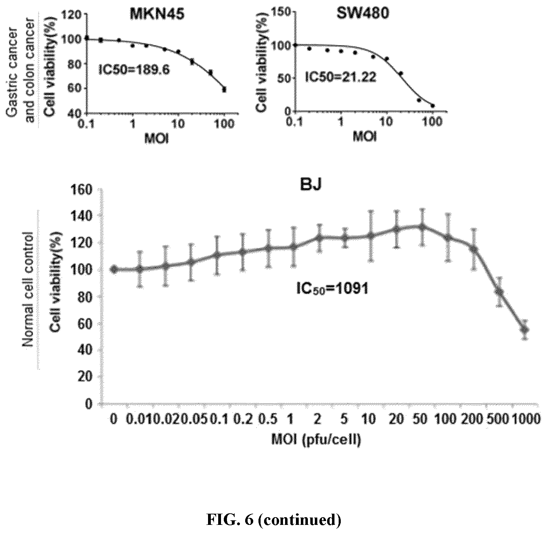

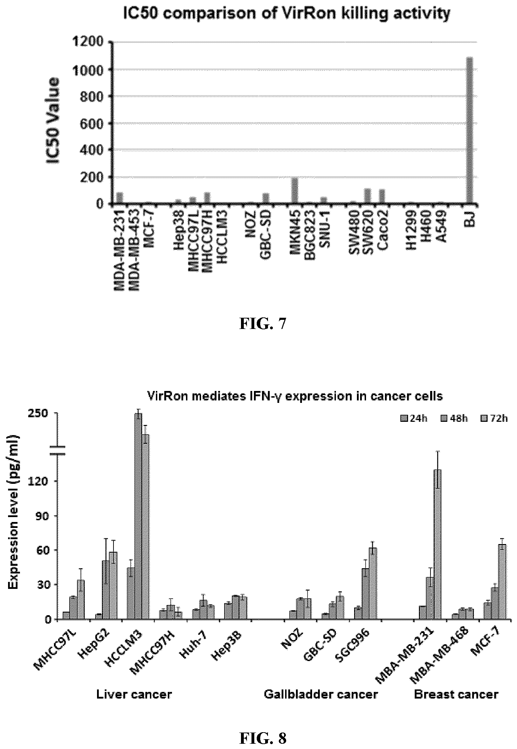

The specific killing and inhibition on tumor cells and normal cells by oncolytic adenovirus VirRon was detected by the CCK8 experiment. The tumor cell lines and normal cell lines in the logarithmic growth phase were collected and plated on a 96-well plate at 1×10⁴ cells/well. After the cells adhered to the wall, serum-free culture medium was used instead. VirRon was added at a gradient MOI for cell infection, and 8 replicate wells were set for each MOI value. Cells were cultured in an incubator for 2 h. Then, the serum culture medium was used instead at 100 µl/well. After 48 h of culture, the culture medium was discarded, and CCK8 solution was added. Cells were then placed in the incubator and incubated for 4 h. The light absorption value at 490 nm wavelength was measured with a microplate reader. The cell survival rate was calculated to plot the cell survival curve. The results showed that VirRon had obvious killing and inhibiting effects on a variety of solid tumor cells, and was closely related to the intensity of viral infection. Different cell lines of the same type of tumor had different sensitivities to VirRon, and the killing IC50 value of VirRon against lung cancer H460 cells was only 6.656 pfu/cell. VirRon showed no obvious killing activity against normal fibroblast BJ cells, with an IC50 value up to 1091 pfu/cell (FIG. 6 and FIG. 7, Table 1).

**Table 1. Killing effect of oncolytic adenovirus VirRon on cultured cells (IC50 comparison)**

| | **Cell lines** | **IC50 (pfu/cell)** |
|---|---|---|
| Breast cancer | MDA-MB-231 | 82.04 |
| | MDA-MB-453 | 10.52 |
| | MCF-7 | 16.72 |
| Liver cancer | Hep3B | 28.94 |
| | MHCC97L | 46.43 |
| | MHCC97H | 82.09 |
| | HCCLM3 | 9.85 |
| Gallbladder cancer | NOZ | 16.01 |
| | GBC-SD | 79.41 |
| Gastric cancer | MKN45 | 189.6 |
| | BGC823 | 11.40 |
| | SNU-1 | 47.23 |
| Colon cancer | SW480 | 21.22 |
| | SW620 | 113.4 |
| | Caco2 | 104.7 |
| Lung cancer | H1299 | 12.65 |
| | H460 | 6.66 |
| | A549 | 16.66 |
| Normal cells | BJ | 1091 |

### (3) Oncolytic adenovirus VirRon mediates the expression of anti-cancer genes

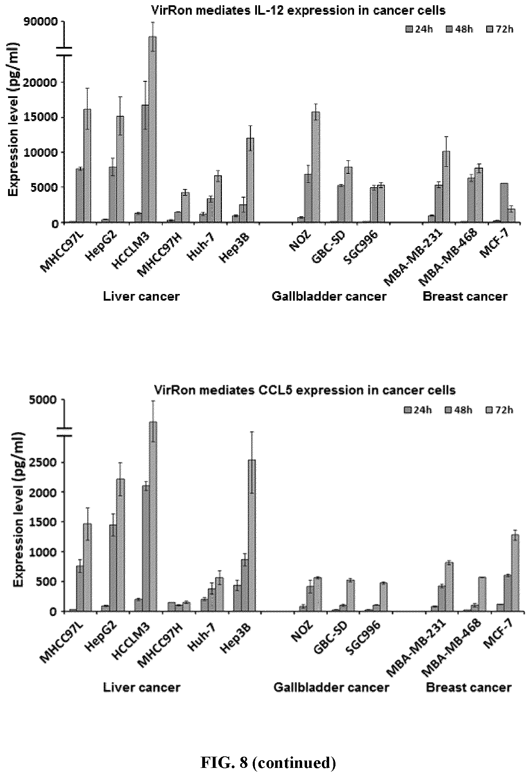

The solid tumor cell lines and normal cell lines in the logarithmic growth phase were collected and plated on a 6-well plate at 1 ×10⁶ cells/well. After the cells adhered to the wall at 24 h, serum-free culture medium was used instead. VirRon was added at MOI = 5 pfu/cell for cell infection. After culturing for 24 h, 48 h, and 72 h, the supernatant was collected, and the protein expression of IL-12, IFN-γ and CCL5 genes was detected by ELISA. The results showed that VirRon can mediate the high-efficiency expression of IFN-γ, IL-12 and CCL5 proteins, and the expression levels gradually increased over time (FIG. 8).

### (4) Effects of the arrangement and connection order of anti-cancer genes in oncolytic adenovirus on packaging efficiency and anti-cancer activity

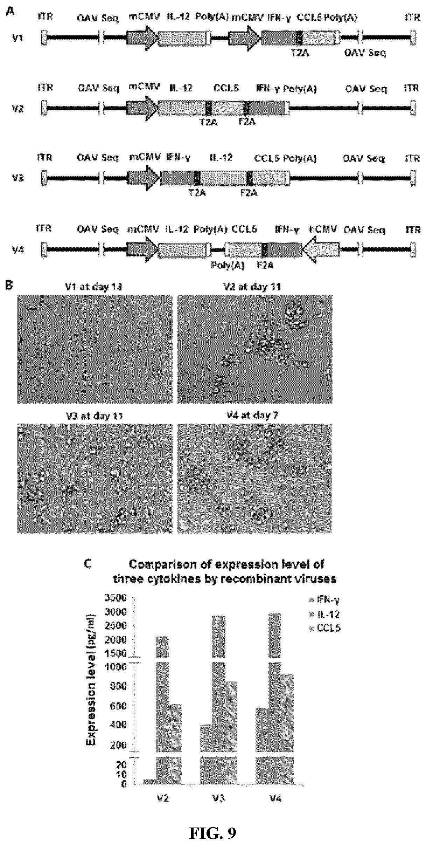

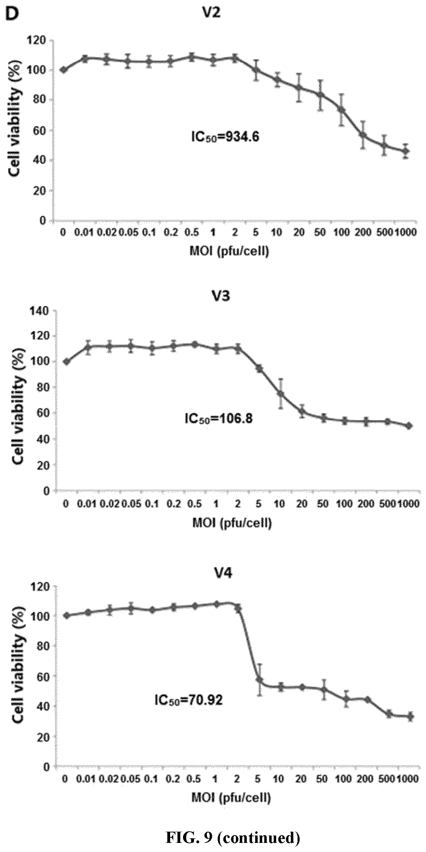

Oncolytic adenovirus VirRon was loaded with three anti-cancer immune factors IL-12, IFN-γ and CCL5. The arrangement and connection order of the three factor genes had a significant impact on the success rate of virus packaging and anti-cancer activity. The shuttle plasmids (V1-V4) with three factors in different arrangements were designed, constructed, and recombined with the adenovirus backbone plasmid, and then the oncolytic adenoviruses were packaged in HEK293 cells (FIG. 9A). The results showed that for V1, no plaque lesions appeared on the cells until Day 13, indicating that the virus was not successfully packaged. Plaques appeared on Day 11 for V2 and V3 and on Day 7 for V4, indicating successful virus recombination (FIG. 9B). The cell supernatant was collected 48 h after the plaque appeared, and the expression of the three factors was detected by ELISA. V2, V3, and V4 expressed high levels of IL-12 and CCL5, but V2 expressed extremely low IFN-γ expression, which was significantly lower than that of V3 and V4 (FIG. 9C). Lung cancer A549 cells were cultured and infected with V2, V3, and V4 viruses at gradient infection intensities (MOI=0.01-1000 pfu/cell). The effect of the virus on cell proliferation activity at 72 h was detected by a CCK-8 kit, confirming that V4 had the most obvious inhibitory effect on A549 cells (FIG. 9D). It was finally determined that the arrangement and connection order of the three factors in V4 (i.e. VirRon) was not only beneficial to the recombinant packaging and gene expression of the virus, but also conducive to improving the anti-cancer activity of the virus.

### (5) Effects of combinations of chimeric Hexon (Ad5H48) fragments in oncolytic adenovirus on packaging and amplification efficiency

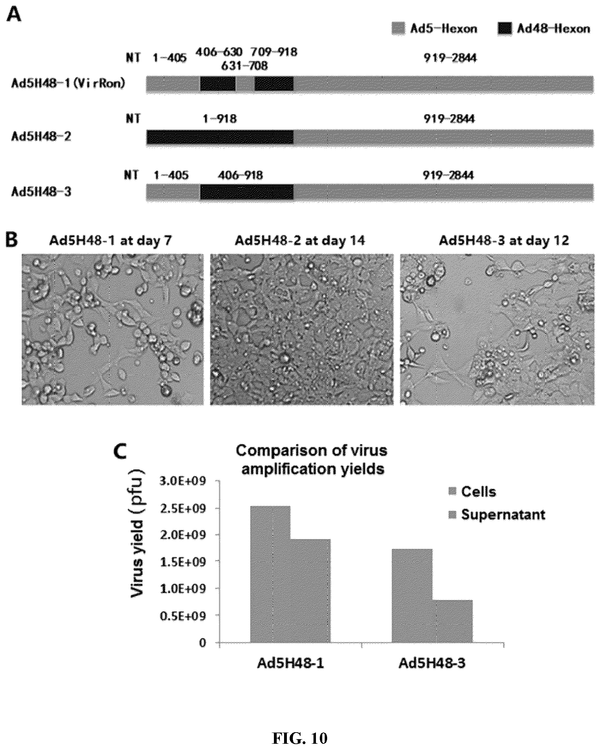

The capsid Hexon (Ad5H48) of oncolytic adenovirus VirRon is a chimera of the Hexon sequences of two serotype viruses, Ad5 and Ad48. The purpose is to avoid the interception of pre-existing Ad5 virus neutralizing antibodies in the body and the adsorption of the virus by the liver. The full length of Ad5H48 encoding cDNA is 2844 bp. We designed chimeric methods with three different fragment combinations to perform viral recombinant packaging and amplification in HEK293 cells (FIG. 10A). The experiment found that Ad5H48-1 (i.e. VirRon) appeared plaques on the cells on the 7th day, Ad5H48-2 showed no plaque lesions until the 14th day during the observation, and Ad5H48-3 showed plaques on the 12th day, confirming that Ad5H48-1 (i.e. VirRon) produced the virus the fastest (FIG. 10B). HEK293 cells were infected with Ad5H48-1 and Ad5H48-3 at MOI=10 pfu/cell. After 96 h, the cells and supernatant were collected respectively. The virus titer was detected by the TCID50 method, and the results showed that the titer of Ad5H48-1 in both the cells and the supernatant was higher than that of Ad5H48-3 (FIG. 10C). It was finally confirmed that Ad5H48-1 (i.e. VirRon) had the best packaging and amplification efficiency.

### Example 3 Animal experiments using VirRon, the chimeric broad-spectrum oncolytic adenovirus for synergistic and enhanced immunotherapy via multiple mechanisms of the present disclosure

### (1) Animal model experiments of VirRon against liver cancer

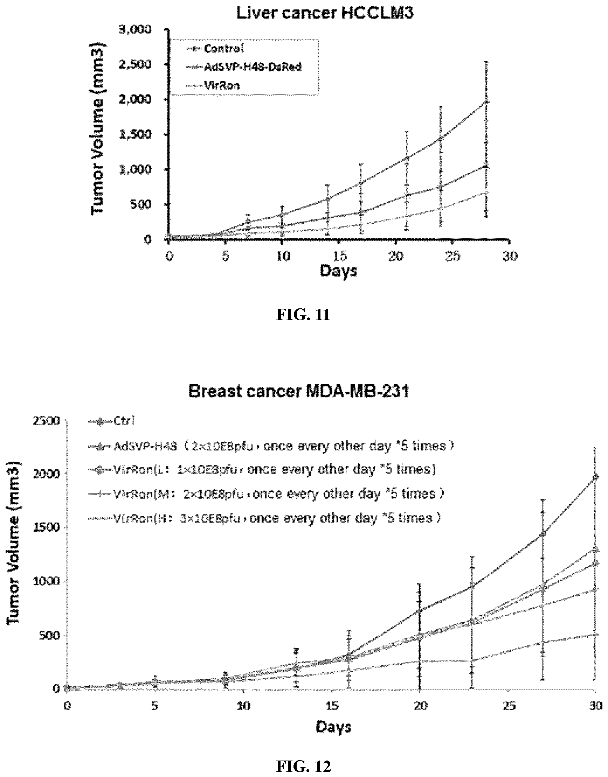

Thirty healthy purebred BALB/C nude mice, 4 weeks old, male, were provided by Chengqin Biotechnology (Shanghai) Co., Ltd., certificate number SCXK (Shanghai) 2012-0002. The suspension of liver cancer HCCLM3 cells in the logarithmic growth phase was injected subcutaneously into the right axilla of nude mice at a concentration of 5 ×10⁶ cells/100 µl/mouse. Twelve days after inoculation, the tumor formation rate was 100%, and the average of the maximum and minimum diameter of the transplanted tumors was approximately 5.0 mm. The mice were randomly divided into 3 groups (VirRon group, AdSVP-H48-DsRed group, and blank control group), with 10 animals in each group. In each group, one mouse with the largest tumor and one mouse with the smallest tumor were first eliminated, and the remaining 8 mice were put into experimental therapeutic observation. The two virus treatment groups were given multiple intratumoral injections of the corresponding adenovirus, with a dose of 3×10⁸ pfu/100 µl per animal each time, once every other day, for a total of 5 times. The blank control group was simultaneously given virus preservation solution, 100 µl per animal each time. After treatment, the tumor size was measured regularly to calculate the tumor volume using the formula of "maximum diameter × minimum diameter² ×0.5", and a growth curve was plotted. During the experiment, if the tumor volume in any group exceeded the upper limit of 2000 mm³ allowed by the Animal Experiment Ethics Committee, the experimental observation will be terminated. The results showed that from the experimental observation to 28 days after treatment, the tumor inhibition rate of the VirRon treatment group was 65.51%, which was significantly different from the control group (P=0.0001). The control virus was AdSVP-H48-DsRed, which did not carry a therapeutic gene but a red fluorescent reporter gene. It also showed significant oncolysis, with a tumor inhibition rate of 45.91%, and the difference was statistically significant compared with the control group (P=0.01) (FIG. 11).

### (2) Animal model experiments using VirRon against breast cancer

Thirty healthy purebred BALB/C nude mice, 4 weeks old, male, were provided by Chengqin Biotechnology (Shanghai) Co., Ltd., certificate number SCXK (Shanghai) 2012-0002. The suspension of breast cancer MDA-MB-231 cells in the logarithmic growth phase was injected subcutaneously into the right axilla of nude mice at a concentration of 5×10⁶ cells/100 µl/mouse. Ten days after inoculation, the tumor formation rate was 100%, and the average of the maximum and minimum diameter of the transplanted tumors was approximately 3.0 mm. The three animals with the largest tumors and the 2 animals with the smallest tumors were eliminated, and the remaining 25 animals were randomly divided into 5 groups (VirRon low-dose group, VirRon medium-dose group, VirRon high-dose group, AdSVP-H48 group, and blank control group), with 5 animals in each group. The virus treatment groups were given multiple intratumoral injections of the corresponding adenovirus. The high-dose group was given a dose of 3×10⁸ pfu/100 µl per animal each time, the medium-dose group was given a dose of 2×10⁸ pfu/100 µl per animal each time, and the low-dose group was given a dose of 1 ×10⁸ pfu/100 µl per animal each time, once every other day, for a total of 5 times. The AdSVP-H48 group was injected with a medium dose, and the blank control group was simultaneously given virus preservation solution, 100 µl per animal each time. After treatment, the tumor size was measured regularly to calculate the tumor volume using the formula of "maximum diameter × minimum diameter ² ×0.5", and a growth curve was plotted. During the experiment, if the tumor volume in any group exceeded the upper limit of 2000 mm³ allowed by the Animal Experiment Ethics Committee, the experimental observation will be terminated. The results showed that from the experimental observation to 30 days after treatment, the tumor inhibition rates of the VirRon high-, medium-, and low-dose groups were 74.15%, 52.80%, and 40.66 % respectively. The control virus AdSVP-H48 that did not carry a therapeutic gene had a tumor inhibition rate of only 33.38% (FIG. 12).

### (3) Animal model experiments using VirRon against gallbladder cancer

Gallbladder cancer is highly malignant and progresses rapidly, and there is no clinical treatment except surgery. By establishing a rapidly growing transplanted tumor model of SGC-996 cells, the efficacy and synergistic mechanism of VirRon treatment were observed.

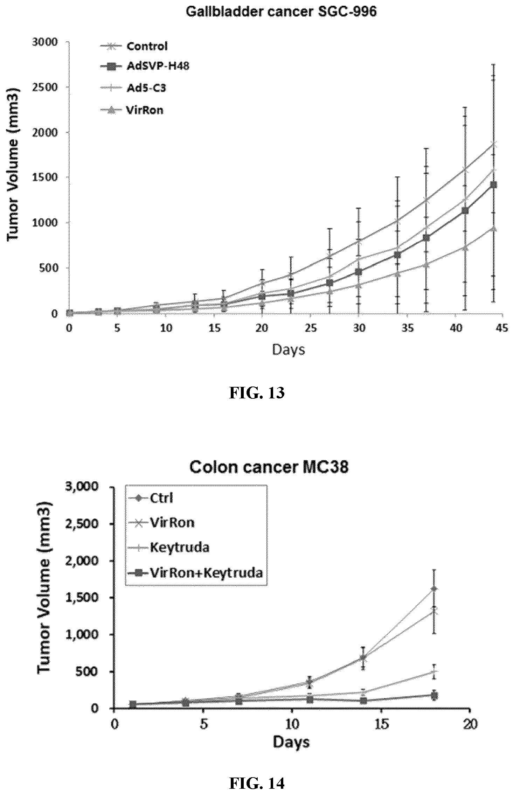

Forty healthy purebred BALB/C nude mice, 4 weeks old, male, were provided by Chengqin Biotechnology (Shanghai) Co., Ltd., certificate number SCXK (Shanghai) 2012-0002. The suspension of gallbladder cancer SGC-996 cells in the logarithmic growth phase was injected subcutaneously into the right axilla of nude mice at a concentration of 5×10⁶ cells/100 µl/mouse. 14 days after inoculation, the tumor formation rate was 100%, and the average of the maximum and minimum diameter of the transplanted tumors was approximately 5.0 mm. They were randomly divided into 4 groups (VirRon group, AdSVP-H48 control virus group, Ad5-C3 non-replicating virus group, and blank control group), with 10 animals in each group. In each group, one mouse with the largest tumor was first eliminated, and the remaining 9 mice were put into experimental therapeutic observation. The VirRon group, AdSVP-H48 group and Ad5-C3 group were given multiple intratumoral injections of the corresponding virus, with a dose of 2×10⁸ pfu /100µl per animal each time, once every other day, for a total of 5 times. The blank control group was simultaneously given virus preservation solution, 100 µl per animal each time. After treatment, the tumor size was measured regularly to calculate the tumor volume using the formula of "maximum diameter × minimum diameter² ×0.5", and a growth curve was plotted. The results showed that from the experimental observation to 44 days after treatment, the tumor inhibition rate of the VirRon treatment group was 49.47%, which was significantly different from the control group (P=0.0241). The empty control oncolytic adenovirus AdSVP-H48 that did not carry a therapeutic gene and the non-proliferating control adenovirus Ad5-C3 expressing three cytokines IL-12, IFN-γ, and CCL5 had a tumor inhibition rate of only 24.03% and 15.26%, respectively. Statistics showed that the Q value of drug synergy was 1.39, confirming that VirRon exerted a synergistic effect on the oncolytic effect of AdSVP-H48 virus and the anti-cancer effect of cytokines (FIG. 13).

### (4) Combination of VirRon and PD-1 antibody for treating colon cancer transplanted tumors in humanized mouse model

46 healthy C57-hPD1 humanized mice, female, 5-6 weeks old, purchased from Biocytogen Biotechnology (Beijing) Co., Ltd., were inoculated subcutaneously on the right back with colon cancer cell line MC38 cells at 1 ×10⁶ cells/animal. When the average tumor size reached 50-80 mm³, the three animals with the largest tumors and the three animals with the smallest tumors were eliminated, and the remaining 40 animals were randomly divided into 4 groups (blank control, VirRon alone, Keytruda alone, and combination groups) and administered for treatment. VirRon was injected into the tumor at multiple points, with a dose of 3×10⁸ pfu/100 µl per animal each time, once every other day, for a total of 5 times. Keytruda was injected intraperitoneally, with a dose of 2.5 mg/kg per animal each time, twice a week, for a total of 6 times. The blank control group was simultaneously given virus preservation solution, 100 µl per animal each time. After treatment, the tumor size was measured regularly to calculate the tumor volume using the formula of "maximum diameter × minimum diameter² ×0.5", and a growth curve was plotted. By 18 days after the first treatment, the tumor inhibition rates of the VirRon alone group, Keytruda alone group, and combination group were 18.71%, 69.05%, and 88.86%, respectively (FIG. 14). There were significant differences between the VirRon + Keytruda combination group and the VirRon alone group, the Keytruda alone group, and the control group (P=0.0013, P= 0.0110, P=0.0004). Statistics showed that the Q value of drug synergy was 1.19, confirming that the combination of VirRon and Keytruda had a synergistic effect.

### (5) Combination of VirRon and CAR-T cells for treating prostate cancer transplanted tumors in NCG mouse model

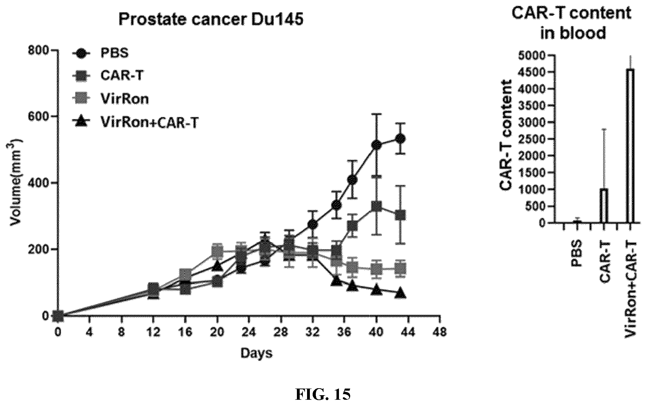

CAR-T cells were prepared by the Cancer Institute of Xuzhou Medical University with a target of B7-H3. Nineteen healthy NCG mice, male, 4-5 weeks old, purchased from Gempharmatech (Jiangsu) Co., Ltd., were inoculated subcutaneously on the right back with prostate cancer cell line Du145 cells at 3 ×10⁶ cells/mouse. When the average tumor size reached about 50 mm³, mice were randomly divided into 4 groups (blank control, VirRon alone, CAR-T alone, and combination groups), with 5 animals in each group (4 animals in the CAR-T alone group) and administered for treatment. The total dose of VirRon per mouse was 1 ×10⁹ pfu, which was divided into 3 intratumoral injections, once every other day, for a total of 5 times. On the second day after completing the virus injection, CAR-T was injected into the tail vein at 2×10⁶ cells/mouse. The blank control group was given PBS buffer simultaneously. After treatment, the tumor size was measured regularly to calculate the tumor volume using the formula of "maximum diameter × minimum diameter² ×0.5", and a growth curve was plotted. By 43 days after the first treatment, the tumor inhibition rates of the VirRon alone group and the CAR-T alone group were 80.19% and 48.83%, respectively, and the tumor inhibition rate of the combination group was as high as 97.56%. There were significant differences between the VirRon +CAR-T combination group and the VirRon alone group, the CAR-T alone group, and the control group (P=0.0008, P=0.0000, P=0.0000) (FIG. 15, left), confirming that the combination of VirRon and CAR-T can improve the efficacy. At the end of the observation period, the blood of the mice was extracted, and the CAR-T cell content was detected by flow cytometry. The results showed that, when VirRon was combined with CAR-T, VirRon can significantly extend the survival time of CAR-T and increase the number of CAR-T cells in the blood (FIG. 15, right).

The above are only the preferred embodiments of the present disclosure. It should be pointed out that those of ordinary skill in the art can also make several improvements and supplements without departing from the method of the present disclosure, and these improvements and supplements should also be regarded within the protection scope of the present disclosure.

## Claims

1. A chimeric broad-spectrum oncolytic adenovirus for synergistic and enhanced immunotherapy via multiple mechanisms, wherein the oncolytic adenovirus simultaneously expresses three factors, IL-12, IFN-γ and CCL5; capsid protein Hexon of the oncolytic adenovirus is a chimera of Hexon sequences of two serotype viruses Ad5 and Ad48; Fiber protein of the oncolytic adenovirus is a chimera of Fiber sequences of two serotype viruses Ad5 and Ad11; the oncolytic adenovirus comprises a tumor-specific strong promoter and an oxygen-dependent element switch (oxygen-dependent degradation domain, ODD), and Elb-55kD gene is deleted, E1a-CR2, E1b-19kD and part of the protein coding sequence in E3 region are knocked out.

2. The chimeric broad-spectrum oncolytic adenovirus according to claim 1, wherein in the genome of the oncolytic adenovirus, cDNA sequences of IFN-γ and CCL5 genes are in one expression cassette driven by human cytomegalovirus (hCMV) promoter, and cDNA sequence of IL-12 gene is in one expression cassette driven by murine cytomegalovirus (mCMV) promoter, and the two expression cassettes are arranged "foot to foot".

3. The chimeric broad-spectrum oncolytic adenovirus according to claim 2, wherein a sequence comprising the two expression cassettes simultaneously expressing three factors, IL-12, IFN-γ and CCL5 and the promoters is set forth in 29422-33108 bp of SEQ ID NO: 3.

4. The chimeric broad-spectrum oncolytic adenovirus according to claim 1, wherein a coding sequence of the capsid protein Hexon of the oncolytic adenovirus is set forth in 18327-21170 bp of SEQ ID NO: 3.

5. The chimeric broad-spectrum oncolytic adenovirus according to claim 1, wherein a coding sequence of the Fiber protein of the oncolytic adenovirus is set forth in 33373-34356 bp of SEQ ID NO: 3.

6. The chimeric broad-spectrum oncolytic adenovirus according to claim 1, wherein an entire genome sequence of the oncolytic adenovirus is set forth in SEQ ID NO: 3.

7. An oncolytic adenovirus simultaneously expressing three factors, IL-12, IFN-γ and CCL5, wherein in the genome of the oncolytic adenovirus, cDNA sequences of IFN-γ and CCL5 genes are in one expression cassette driven by human cytomegalovirus (hCMV) promoter, and cDNA sequence of IL-12 gene is in one expression cassette driven by the murine cytomegalovirus (mCMV) promoter, and the two expression cassettes are arranged "foot to foot".

8. The oncolytic adenovirus simultaneously expressing three factors, IL-12, IFN-γ and CCL5 according to claim 7, wherein a sequence comprising the two expression cassettes simultaneously expressing three factors, IL-12, IFN-γ and CCL5 and the promoters is set forth in 29422-33108 bp of SEQ ID NO: 3.

9. Use of the chimeric broad-spectrum oncolytic adenovirus according to claim 1 in the manufacture of a medicament for the treatment and/or prevention and/or auxiliary treatment of a cancer or tumor.

10. The use according to claim 9, wherein the cancer or tumor is selected from the group consisting of breast cancer, liver cancer, gallbladder cancer, gastric cancer, colon cancer, lung cancer, prostate cancer, lymphoma, colorectal cancer, ovarian cancer, cervical cancer, bile duct cancer, esophageal cancer, kidney cancer, glioma, melanoma, pancreatic cancer, bladder cancer and head and neck cancer.
